# NEUE EUROPÄISCHE PATENTSCHRIFT

(11) **EP 2 434 202 B2**
(45) Veröffentlichungstag und Bekanntmachung des Hinweises auf die Entscheidung über den Einspruch: **21.04.2021**
(45) Hinweis auf die Patenterteilung: 02.11.2016
(21) Anmeldenummer: 10181051.3
(22) Anmeldetag: 28.09.2010
(51) Int. Cl.: F21S 8/00, F21V 21/40, F21V 23/04, F21W 131/205

(54) **Operationsleuchte mit steriler Bedieneinrichtung**
Operating light with sterile operating device
Lampe d'opération dotée d'un dispositif de commande stérile

(43) Veröffentlichungstag der Anmeldung: 28.03.2012
(73) Patentinhaber: TRUMPF Medizin Systeme GmbH + Co. KG, 07318 Saalfeld (DE)
(72) Erfinder: Schmid, Michael, 82194, Gröbenzell (DE); Frenzel, Mathias, 82110, Germering (DE); Marka, Rudolf, 85737, Ismaning (DE); Thiessen, Klaus, 82178, Puchheim (DE)
(74) Vertreter: Prüfer & Partner mbB Patentanwälte · Rechtsanwälte

(56) Entgegenhaltungen:
- EP-A1- 2 065 634
- EP-A1- 2 169 965
- WO-A1-96/06516
- WO-A1-2009/052655
- WO-A1-2009/059464
- WO-A1-2010/146446
- WO-A2-2004/080291
- WO-A2-2007/011646
- DE-A1-102008 039 772
- DE-U1- 20 316 755
- FR-A1- 2 557 711
- GB-A- 2 423 378
- US-A1- 2003 185 009
- US-A1- 2008 311 993

## Beschreibung

Die Erfindung bezieht sich auf eine Operationsleuchte mit einer sterilen Bedieneinrichtung, deren Funktionen von einem Operateur steril bedient werden können.

Während Operationen am menschlichen Körper ist es auf Grund der Infektionsgefahr für den Patienten wichtig, dass die Hände des Operateurs oder der Operateure nicht durch Berühren von nicht-sterilisierten Oberflächen unsteril werden.

Um Operationen unter für den Operateur günstigen Bedingungen durchzuführen, ist unter anderem eine gute Ausleuchtung des Operationsfelds erforderlich. Dies kann üblicherweise durch Einstellen verschiedener Parameter der Operationsleuchte erreicht werden. Dazu sind in der Regel eine Position und eine Orientierung des Leuchtenkörpers, eine Fokussierung der Lichtstrahlen auf die Operationsstelle und eine Intensität der abgestrahlten Lichtstrahlen, also die Helligkeit der Beleuchtung der Operationsstelle, einstellbar. Ein Verändern der Position und der Orientierung wird üblicherweise durch den Operateur selbst durchgeführt, der dazu den Leuchtenkörper an einem sterilen Handgriff, der über eine Handgriffaufnahme des Leuchtenkörpers geschoben wird, nimmt, und an die gewünschte Position und in die gewünschte Orientierung schwenkt. Durch Verdrehen des sterilen Handgriffs wird dann üblicherweise die Fokussierung der Lichtstrahlen, d.h. der Abstand des Schnittpunkts der abgestrahlten Lichtstrahlen von dem Leuchtenkörper eingestellt.

Die Offenlegungsschrift der Patentanmeldung GB 2 423 378 A zeigt eine Lampe, die einen Leuchtenkörper und daran angeordnete Sensoren aufweist, die berührungslos eine Position z.B. einer Hand relativ zu dem Leuchtenkörper erfassen. Abhängig von der Position der Hand relativ zu dem Leuchtenkörper wird der Leuchtenkörper bewegt, um einen konstanten Abstand zwischen der Handfläche und den Sensoren beizubehalten. Dabei wird die Richtung des von dem Leuchtenkörper ausgestrahlten Lichts eingestellt.

Ein Einstellen der Helligkeit wird normalerweise mit Hilfe von nicht-sterilisierten Bedienelementen am Rand des Leuchtenkörpers, am Tragsystem, oder an einer Wandbedieneinheit nicht durch den Operateur durchgeführt, sondern von Operationspersonal, bei dem geringere Anforderungen hinsichtlich Sterilität bestehen. Hierzu ist aber zusätzliches Personal erforderlich und eine wunschgemäße Einstellung der Helligkeit ist durch die notwendige Kommunikation zwischen Operateur und Personal aufwendiger, was besonders in kritischen Operationssituationen für den Patienten gefährlich werden kann.

Um dieses Problem zu umgehen, ist z.B. aus der EP-A-1 750 052 ein sterilisierbares Bedienelement bekannt, das zusätzlich zu dem sterilisierbaren Handgriff am Leuchtenkörper angebracht ist, wodurch auch weitere Funktionen, z.B. Einstellen einer Farbtemperatur, Einstellen einer Lichtverteilung oder Bedienen einer Kamera, steril bedient werden können. Hierbei ist jedoch ein weiteres Bedienelement erforderlich, und durch die Möglichkeit, Einstellungen in mehreren Betriebsmodi durchzuführen, muss der Operateur möglicherweise erst den gewünschten Betriebsmodus auswählen. Somit ist diese Einstellmöglichkeit bei einer Operationsleuchte, die nur die Funktionen Helligkeit und Fokussierung einstellbar hat, aufwendig und lenkt den Operateur ab.

Problematisch ist außerdem, dass bei einem Einstellen der Orientierung der Operationsleuchte durch den Operateur, wenn der Leuchtenkörper um seine Achse gedreht wird, diese Drehung am sterilen Handgriff erfolgt, und dadurch auch die Fokussierung der Lichtstrahlen verändert wird.

Daraus ergibt sich die Aufgabe, eine Operationsleuchte bereitzustellen, die eine Veränderung der Orientierung der Operationsleuchte für den Operateur unter sterilen Bedingungen ermöglicht, ohne die Fokussierung zu verändern, und auch die anderen vorgenannten Probleme löst.

Die Aufgabe wird durch die Merkmale des Anspruchs 1 gelöst. Weiterentwicklungen der Erfindung sind Gegenstand der abhängigen Ansprüche.

Ein Leuchtenkörper ist dazu so ausgebildet, dass er eine Bedieneinrichtung aufweist, die berührungslos betätigt werden kann. Berührungslos heißt in diesem Zusammenhang, dass zwar die Bedieneinrichtung selbst, insbesondere ein Sensor der Bedieneinrichtung, nicht berührt wird, sterile Bauteile des Leuchtenkörpers, die den Sensor der Bedieneinrichtung abdecken, jedoch berührt werden können.

Die Erfindung wird nun unter Bezugnahme auf die beigefügten Zeichnungen anhand eines Ausführungsbeispiels näher erläutert.

Im Einzelnen zeigen:
- Fig. 1: eine perspektivische Darstellung einer Operationsleuchte,
- Fig. 2: einen Leuchtenkörper der Operationsleuchte von Fig. 1 in zwei verschiedenen Fokussiereinstellungen,
- Fig. 3: eine Handgriffaufnahme der Operationsleuchte von Fig. 1 mit einem Handgriff, und
- Fig. 4: eine Bedienfolie einer Bedieneinrichtung der Operationsleuchte von Fig. 1.

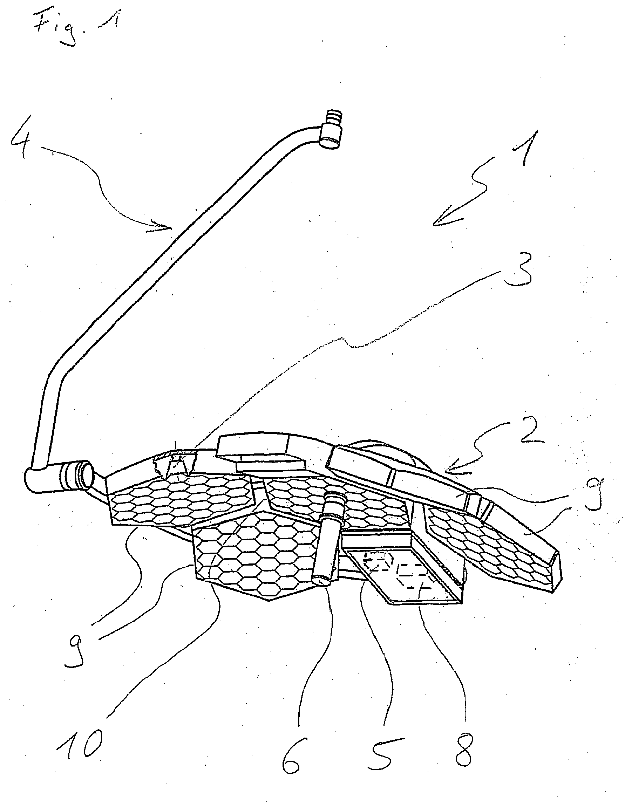

Fig. 1 zeigt eine perspektivische Darstellung einer Operationsleuchte 1, die einen Leuchtenkörper 2 aufweist. Der Leuchtenkörper 2 ist über eine Aufhängevorrichtung 4, von der nur ein unterer Abschnitt gezeigt ist, mit einer nicht gezeigten Raumdecke,
einem fahrbaren Stativ, oder ähnlichem verbunden. Die Aufhängevorrichtung 4 stellt eine kardanische Aufhängung dar, so dass der Leuchtenkörper 2 innerhalb eines bestimmten Aktionsradius in einer beliebigen Position und in einer beliebigen Orientierung positioniert werden kann.

Der Leuchtenkörper 2 weist in dieser Ausführungsform vier Randmodule 9 und ein Zentralmodul 10 auf, wobei die Randmodule 9 schwenkbar an dem Zentralmodul 10 angebracht sind. Alternativ kann auch eine andere Anzahl von Modulen oder eine unterschiedliche Anordnung der Module vorgesehen sein. In weiteren Ausführungsformen kann die Operationsleuchte 1 anstatt mit Modulen auch als aufgelöstes Lichtsystem mit Einzelscheinwerfern versehen sein, oder als Großspiegelleuchte ausgeführt sein. Darüber hinaus können alternativ schwenkbare Module oder schwenkbare Scheinwerfer innerhalb eines Gehäuses eines Leuchtenkörpers vorgesehen sein.

Die Randmodule 9 und das Zentralmodul 10 weisen jeweils eine Mehrzahl von Lichtquellen 3 auf, die in dieser Ausführungsform jeweils als eine LED mit einer Linse ausgebildet sind. Die Lichtquellen 3 geben jeweils ein Lichtbündel ab. In den alternativen Ausführungsformen haben die Einzelscheinwerfer des aufgelösten Lichtsystems jeweils eine Lichtquelle, und eine Großspiegelleuchte weist eine einzelne betriebene Lichtquelle im Leuchtenkörper auf.

Der Leuchtenkörper 2 ist in dieser Ausführungsform mit einer Handgriffaufnahme 6 versehen, die auf der Unterseite des Zentralmoduls 10 an einer Lichtaustrittsfläche vorsteht. Die Handgriffaufnahme 6 kann in alternativen Ausführungsformen auch an einer anderen Stelle am Leuchtenkörper 2 oder an der Aufhängevorrichtung 4 vorgesehen sein.

Die Operationsleuchte 1 weist weiterhin eine Fokussiereinrichtung 5 und eine Dimmvorrichtung 8 auf, die in dieser Ausführungsform in einem Gehäuse des Leuchtenkörpers 2 untergebracht sind, sich jedoch nicht notwendigerweise hier befinden müssen, sondern auch an einer geeigneten Stelle der Operationsleuchte 1 vorgesehen sein können. Die Funktion der Fokussiereinrichtung 5 und der Dimmvorrichtung 8 wird nachstehend erklärt.

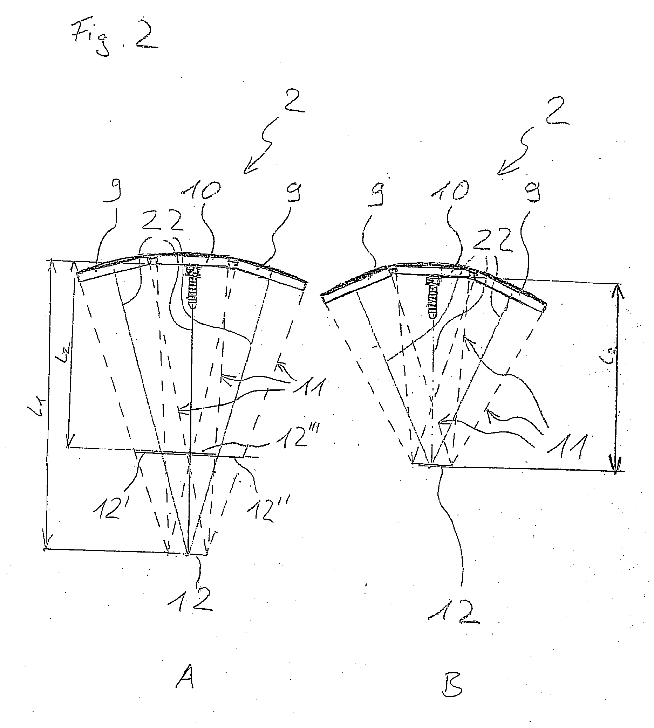

Fig. 2 zeigt den Leuchtenkörper 2 in zwei verschiedenen Fokussiereinstellungen. Die Randmodule 9 und das Zentralmodul 10 geben jeweils ein Lichtbündel 11 aus dem Leuchtenkörper 2 ab, das von den einzelnen Lichtquellen 3 erzeugt wird, wobei die Lichtbündel 11 selbst so angepasst sind, dass jedes einzelne Modul ein Leuchtfeld, d.h. eine beleuchtete Fläche auf der Operationsstelle eines Patienten bildet. Bei geeigneter Fokussierung der Lichtbündel der Module, d.h. wenn die Winkelstellung zwischen dem jeweiligen Randmodul 9 und dem Zentralmodul 10 so gewählt ist, dass die einzelnen Leuchtfelder auf der Operationsstelle zur Deckung kommen, wird ein gemeinsames Leuchtfeld 12 auf der Operationsstelle gebildet. Die Lichtbündel 11 weisen jeweils eine Mittelachse 22 auf, die sich bei der Bildung des gemeinsamen Leuchtfelds 12 auf der beleuchteten Fläche auf der Operationsstelle schneiden. In der Abbildung A schneiden sich die Mittelachsen 22 der Lichtbündel 11 in einem Abstand l₁ von dem Leuchtenkörper 2. In dieser Einstellung ist die Abbildung der Lichtbündel 11 auf der Operationsstelle identisch und das gemeinsame Leuchtfeld 12 wird gebildet. Bei einem Abstand l₂ des Operationsfelds vom Leuchtenkörper 2 werden bei der gleichen Winkeleinstellung der Randmodule 9 zu dem Zentralmodul 10 die Lichtbündel 11 jeweils auf Leuchtfeldern 12', 12'', 12''' abgebildet, die nicht identisch sind, sondern sich nur teilweise überlappen.

Um die Leuchtfelder 12', 12" und 12''' zur Deckung zu bringen, so dass sie identisch auf der Operationsstelle im Abstand l₂ abgebildet werden, ist es erforderlich, die Lichtbündel 11, die aus dem Leuchtenkörper 2 abgegeben werden, zu fokussieren. Wie in Abbildung B gezeigt, werden die Randmodule 9 zu dem Mittelmodul 10 so verschwenkt, dass sich die Mittelachsen 22 der Lichtbündel 11 wieder auf der Operationsstelle im Abstand l₂ schneiden, so dass die Lichtbündel auf dem Operationsfeld in dem gemeinsamen Leuchtfeld 12 abgebildet werden, und somit das Operationsfeld optimal ausleuchten.

Die Randmodule 9 werden durch einen nicht gezeigten Antrieb verschwenkt. Der Antrieb wird über die Fokussiereinrichtung 5 (Fig. 1) gemäß einer Eingabe eines Bedieners angesteuert. Eine Einstellüng des Winkels der Randmodule 9 zu dem Zentralmodul 10 ist zwischen zwei Endstellungen stufenlos möglich. Alle Randmodule 9 werden dabei simultan verstellt, so dass sich der Schnittpunkt der Mittelachsen 22 der Lichtbündel 11 jeweils auf der Mittelachse 22 des Lichtbündels 11 des Zentralmoduls 10 befindet und jeweils einen anderen Abstand zum Leuchtenkörper 2 hat.

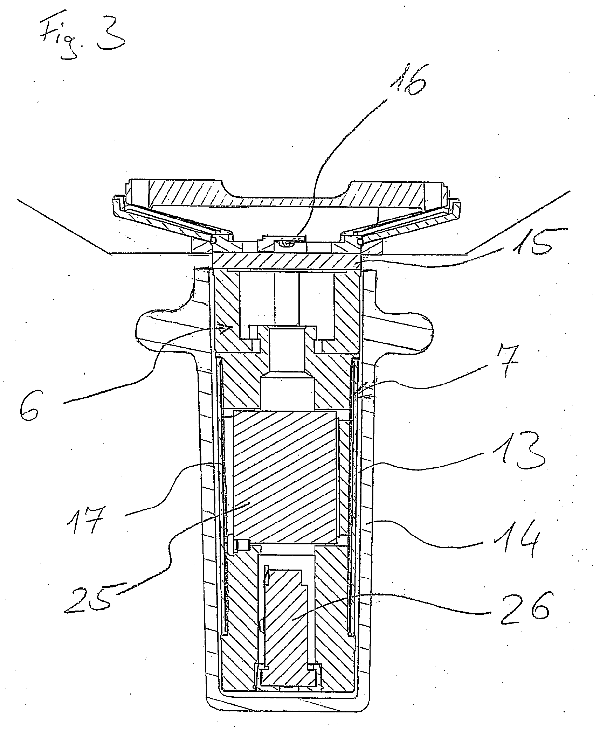

Die Verstellung des Abstands zwischen dem Leuchtenkörper 2 und dem Leuchtfeld 12, das durch die fokussierten Lichtbündel 11 gebildet wird, wird in anderen Ausführungsformen in ähnlicher Weise durch Verstellen der Abstrahlrichtung von Modulen oder Einzelscheinwerfern, die das aufgelöste Lichtsystem bilden, oder die innerhalb des Leuchtenkörpers vorgesehen sind, oder, bei Großspiegelleuchten mit einem Leuchtmittel und einem Reflektor, durch Verlagern des Leuchtmittels auf der Symmetrieachse des Reflektors um den Brennpunkt des Reflektors herum, ausgeführt. Fig. 3 zeigt die Handgriffaufnahme 6 mit einem um die Handgriffaufnahme 6 angeordneten Handgriff 14. Die Handgriffaufnahme 6 weist eine Bedieneinrichtung 7 auf, die wiederum eine Bedienfolie 17 aufweist. Die Handgriffaufnahme 6 ist in axialer Richtung über eine bestimmte Länge mit einer umlaufenden Vertiefung versehen. In diese Vertiefung ist die Bedienfolie 17 eingelegt, deren Länge in der axialen Richtung der Handgriffaufnahme 6 in etwa der'axialen Länge der Vertiefung entspricht, aber geringfügig kürzer ist, und die eine Breite aufweist, die in etwa dem Umfang der Vertiefung in der Handgriffaufnahme 6 entspricht, so dass der gesamte Umfang der Vertiefung mit der Bedienfolie 17 bedeckt ist. Um die Bedienfolie 17 herum ist eine Schutzabdeckung 13 aus einem Gummimaterial gestülpt. Die Enden der Schutzabdeckung 13 in axialer Richtung bilden jeweils einen nach innen weisenden Kragen, so dass in den sich axial erstreckenden Raum zwischen den Kragen die Bedienfolie 17 aufgenommen werden kann. Damit ist die Bedienfolie 17 vor Feuchtigkeit und mechanischer Beschädigung geschützt.

Die Handgriffaufnahme 6 ist an dem Leuchtenkörper 2 befestigt (Fig. 1). Um die Handgriffaufnahme 6 ist der Handgriff 14 aufgeschoben und mit einem nicht gezeigten Rastelement axial und radial befestigt. Der Handgriff 14 ist aus einem sterilisierbaren Material hergestellt, um so die hygienischen Anforderungen an ein steriles Betätigungselement zu erfüllen.

In alternativen Ausführungsformen kann der Handgriff auch als ein Einmalhandgriff in Form eines sterilen elastischen Kunststoffüberzugs ausgeführt sein.

In der Handgriffaufnahme 6 sind weiterhin eine Auswerteeinheit 25 der Bedienvorrichtung 7, und ein Lasersensor 26 zur Abstandsmessung angeordnet. Diese Bauteile können in alternativen Ausführungsformen auch an anderen Stellen des Leuchtenkörpers 2 oder der Operationsleuchte 1 angeordnet sein.

Am oberen Ende der Bediengriffaufnahme 6 oberhalb des Handgriffs 14 weist diese eine transparente Abdeckung 15 als eine Streuscheibe und ein Leuchtmittel 16 als Anzeigemittel auf. Das Leuchtmittel 16 zeigt, wie später beschrieben wird, Verstellsituationen der Operationsleuchte 1 an.

Die transparente Abdeckung 15 ist umlaufend aus einem transparenten Material angefertigt, so dass die Anzeige des Leuchtmittels 16 aus jeder Richtung um die Operationsleuchte herum wahrgenommen werden kann.

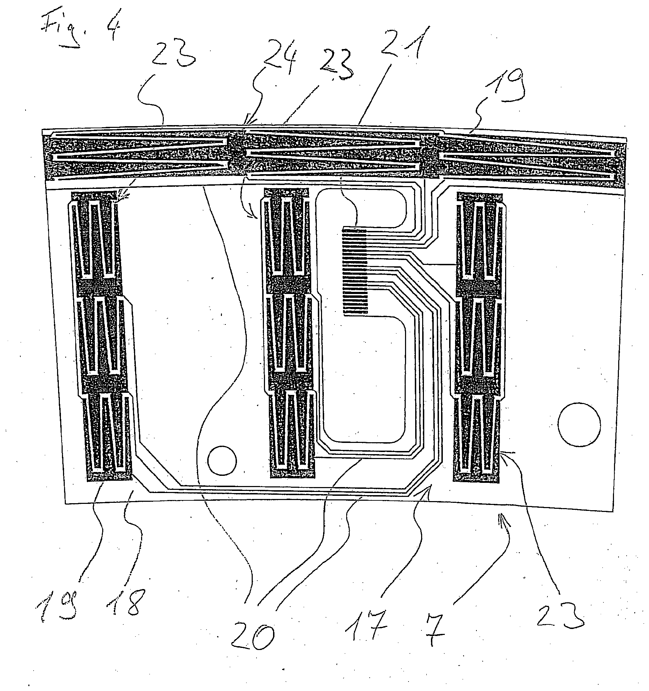

Fig. 4 zeigt die Bedienfolie 17 der Bedieneinrichtung 7. Die Bedienfolie 17 weist, wie bereits in der Beschreibung zu Fig. 3 erklärt, eine Breite auf, die in etwa dem Umfang der Vertiefung der Handgriffaufnahme 6 entspricht. Da die Handgriffaufnahme 6 nicht zylindrisch, sondern leicht konisch ist, ist auch die Breite der Bedienfolie 17 nach unten leicht zulaufend. Die Bedienfolie 17 besteht aus einer Trägerfolie 18, auf der Kupferbahnen 19 aufgebracht sind.

Auf der Bedienfolie 17 sind vier Anordnungen von den Kupferbahnen 19 gezeigt, wobei sich drei Anordnungen jeweils in vertikaler Richtung erstrecken und jeweils einen vertikalen Sensor 23 bilden, und eine Anordnung sich in horizontaler Richtung am oberen Ende der Bedienfolie 17 erstreckt und einen horizontalen Sensor 24 bildet. Die Kupferbahnen 19 sind jeweils über Leiterbahnen 20 mit einem Steckverbinder 21 verbunden. Über den Steckverbinder 21 ist die Bedienfolie 17 mit der Auswerteeinheit 25 der Bedieneinrichtung 7 verbunden. Die Auswerteeinheit 25 kann entweder als separate Einheit ausgebildet sein, oder alternativ in einer Steuerungsvorrichtung der Operationsleuchte 1 integriert sein.

Im eingebauten Zustand bildet somit der horizontale Sensor 24 eine um die Handgriffaufnahme 6 umlaufende Sensorfläche. Die vertikalen Sensoren 23 bilden jeweils eine Sensorfläche in Achsrichtung der Handgriffaufnahme 6, die in Achsrichtung der Handgriffaufnahme 6 gesehen, in einem Winkelabstand von ca. 120° angeordnet sind.

Die Kupferbahnen 19 bilden durch ihre Form und Anordnung zueinander jeweils kapazitive Sensoren..In alternativen Ausführungsformen können auch andere Bedieneinrichtungen, die berührungslos betätigbar sind, wie z.B. optische Bedieneinrichtungen, verwendet werden.

Die Operationsleuchte 1 ist so konfiguriert, dass der horizontale Sensor 24 für ein Dimmen der Lichtquellen 3 der Operationsleuchte 1 verwendet wird, und die drei vertikalen Sensoren 23 für die Fokussierung der Operationsleuchte 1 verwendet werden, indem die Sensoren 23, 24 jeweils durch einen vorbestimmten Bewegungsablauf eines Bedieners berührungslos betätigt werden.

Die Bedieneinrichtung 7 ist so konfiguriert, dass zu einer Ansteuerung der Dimmung, also einer Beleuchtungsstärkeänderung, der horizontale Sensor 24 in einem ersten Schritt eines Dimmverfahrens erfasst, wenn ein Objekt, z.B. ein Finger oder ein Teil eines Operationsbestecks, in den Bereich des horizontalen Sensors 24 gebracht wird. Dadurch gibt der horizontale Sensor 24 ein entsprechendes Signal an die Auswerteeinheit 25 aus, wodurch die Bedieneinrichtung 7 das Einbringen des Objekts in den Bereich des horizontalen Sensors 24 erkennt. Durch Bewegen des Objekts entlang des Umfangs der Handgriffaufnahme 6 oder des montierten Handgriffs 14, also entlang des horizontalen Sensors 24, erfasst der horizontale Sensor 24, in welcher Position sich das Objekt durch die Bewegung relativ zu der ursprünglich erkannten Position befindet, und überträgt entsprechende Signale an die Auswerteeinheit 25. Die Bedieneinrichtung 7 ist so konfiguriert, dass ihre Auswerteeinheit 25 in einem zweiten Schritt erkennt, in welche Richtung das Objekt bewegt wird, nämlich im Uhrzeigersinn oder gegen den Uhrzeigersinn bezüglich der Achse der Handgriffaufnahme 6, und in einem dritten Schritt erkennt, um welche Wegstrecke das Objekt bewegt wird. Die Bedieneinrichtung 7 ist weiterhin so konfiguriert, dass ihre Auswerteeinheit 25 in einem vierten Schritt jeweilige Signale entsprechend der Bewegungsrichtung und der Wegstrecke der Bewegung an die Dimmvorrichtung 8 ausgibt. Eine Bewegungsrichtung im Uhrzeigersinn bezüglich der axialen Richtung der Handgriffaufnahme 6 entspricht hier einer Erhöhung der Helligkeit der Operationsleuchte 1, und eine Bewegung entgegengesetzt dem Uhrzeigersinn entspricht hier einer Verringerung der Helligkeit. Diese Zuordnung kann in anderen Ausführungsformen auch umgekehrt sein.

In dieser Ausführungsform ist die Auswerteeinheit 25 so konfiguriert, dass eine Wegstrecke des Objekts von 1 cm entlang des horizontalen Sensors 24 der Veränderung von einer Helligkeitsstufe entspricht. Die Beleuchtungsstärkeänderung je Helligkeitsstufe ist in der Dimmvorrichtung 8 definiert. In alternativen Ausführungsformen kann die Zuordnung der Wegstrecke zu einer Helligkeitsstufe auch einem anderen Wert entsprechen, oder stufenlos sein.

Die Bedieneinrichtung 7 ist so konfiguriert, dass zu einer Ansteuerung der Fokussierung, einer oder mehrere der vertikalen Sensoren 23 in einem ersten Schritt eines Fokussierverfahrens erkennen, wenn ein Objekt, z.B. ein Finger oder ein Teil eines Operationsbestecks, in den Bereich eines der vertikalen Sensoren 23 gebracht wird/werden, und ein entsprechendes Signal an die Auswerteeinheit 25 ausgibt, wodurch die Bedieneinrichtung 7 das Einbringen des Objekts in den Bereich der vertikalen Sensoren 23 erkennt. Durch Bewegen des Objekts entlang der Handgriffaufnahme 6 in der axialen Richtung der Handgriffaufnahme 6, also entlang der vertikalen Sensoren 23, übertragen die vertikalen Sensoren 23 Signale an die Auswerteeinheit 25, in welcher Position sich das Objekt durch die Bewegung relativ zu der ursprünglich erkannten Position befindet, und die Bedieneinrichtung 7 ist so konfiguriert, dass sie in einem zweiten Schritt erkennt, in welche Richtung, nämlich zum Leuchtenkörper 2 hin oder vom Leuchtenkörper 2 weg, das Objekt bewegt wird. Beim Wegbewegen des Objekts von dem Leuchtenkörper 2 wird durch die Auswerteeinheit 25 der Bedieneinrichtung 7 in einem dritten Schritt ein Signal an die Fokussiereinrichtung 5 gegeben, die Randmodule 9 zusammenzufahren, d.h. den Abstand des Punkts, in dem die Achsen 22 der Lichtbündel 11 fokussiert werden, zu dem Leuchtenkörper 2 zu verringern. Eine Bewegung des Objekts in Richtung zum Leuchtenkörper 2 hin ergibt in einem dritten Schritt die Ausgabe eines Signals der Bedieneinrichtung 7 an die Fokussiereinrichtung 5 zum Auseinander fahren der Module, d.h. der Abstand zwischen dem Schnittpunkt der Achsen 22 der Lichtbündel 11 zu dem Leuchtenkörper 2 wird vergrößert. Das Signal wird ausgegeben, solange die Auswerteeinheit 25 in einem vierten Schritt die Anwesenheit des Objekts während oder nach der Bewegung in dem Bereich eines der vertikalen Sensoren 23 erfasst, bis das Objekt von der Sensorfläche entfernt wird. Bei einer Umkehrung der Bewegungsrichtung des Objekts, ohne dass das Objekt aus dem Sensorbereich entfernt wird, wird ein Signal an die Fokussiereinrichtung 5 gegeben, den Abstand zwischen dem Schnittpunkt der Achsen 22 und dem Leuchtenkörper 2 in die entgegen gesetzte Richtung zu verändern, bis das Objekt von der Sensorfläche entfernt wird.

Die Operationsleuchte 1 befindet sich standardmäßig in einem Automatikmodus, in dem durch eine Abstandsmessung mit Hilfe des Lasersensors 26 der Abstand zwischen dem Leuchtenkörper 2 und der Operationsstelle gemessen wird. Damit wird das optimale Leuchtfeld 12 durch die Fokussiereinrichtung 5 eingestellt. Entsprechend seiner Bedürfnisse kann der Operateur jederzeit die Automatik durch Betätigen der Bedieneinrichtung 7 korrigieren. Alternativ kann bei einem Fehlen der Automatikfunktion beim Einschalten der Operationsleuchte 1 der zuletzt eingestellte Abstand beibehalten werden, oder es kann ein standardmäßig vorbestimmter Wert (z.B. der Normwert von einem Meter) eingestellt werden.

Die Bedieneinrichtung 7 ist mit der Steuerungsvorrichtung der Operationsleuchte 1 verbunden, die wiederum mit der Fokussiereinrichtung 5 und der Dimmvorrichtung 8 verbunden ist und diese ansteuert. In alternativen Ausführungsformen ist die Bedieneinrichtung 7 direkt mit der Fokussiereinrichtung 5 und der Dimmvorrichtung 8 verbunden.

Die Zuordnung zwischen den Sensoren 23, 24 und den Funktionen Dimmen und Fokussieren kann in anderen Ausführungsformen auch in anderer Weise festgelegt sein. Auch ist die Art der Erkennung des Bewegungsablaufs, nämlich einerseits der Erkennung der Richtung und der Wegstrecke einer Bewegung, und andererseits der Erkennung der Richtung der Bewegung und die Dauer der Anwesenheit des Objekts, nicht einem bestimmten Sensor oder einer Funktion fest zugeordnet, sondern die Zuordnung kann in einer beliebigen, geeigneten Weise erfolgen.

Durch die berührungslose Erkennung des Objekts kann der Bediener entweder die entsprechende Funktion der Operationsleuchte 1 bedienen, ohne tatsächlich die Operationsleuchte 1 zu berühren, oder er fährt mit dem Objekt entlang einer sterilisierbaren Abdeckung, in dieser Ausführungsform der sterile Handgriff 14, was beides die sterile Bedienung der Operationsleuchte 1 ermöglicht.

An der Auswerteeinheit 25 der Bedieneinrichtung 7 ist eine Empfindlichkeit der kapazitiven Sensoren durch eine Einrichtung zum Einstellen einstellbar bzw. programmierbar. Um eine sichere und genaue Detektierung des Objekts zu gewährleisten, ist eine Kalibriereinrichtung vorgesehen, die die Bedieneinrichtung 7 beim Einschalten automatisch kalibriert, indem erfasst wird, welche Eingangswerte in die Auswerteeinheit 25 beim Einschalten vorliegen. Diese Werte werden als Grenzwerte einer "Nicht-Betätigung" eingestellt, so dass die Annäherung und Bewegung eines Objekts (z.B. des.Fingers) erkannt wird. Somit wird eine Ansteuerung der Fokussierung oder des Dimmens auch ohne Bediengriff oder mit einem beliebigen Bediengriff (Toleranzausgleich), oder mit z.B. dem elastischen Kunststoffüberzug, durchgeführt. Die erfassten Werte werden während des Betriebs zyklisch überprüft, ob sich länger andauernde Veränderungen der Werte ergeben. Dadurch wird beispielsweise ein Aufschieben und Montieren eines Handgriffs 14 erkannt, der beim Einschalten der Operationsleuchte 1 noch nicht angebracht war, und die Werte werden angepasst, so dass eine Erkennung z.B. eines Fingers trotz des aufgeschobenen Handgriffs 14 erfolgt.

Eine unbeabsichtigte Betätigung der Bedieneinrichtung 7 wird durch die folgenden Maßnahmen unterdrückt. In alternativen Ausführungsformen sind nicht beide Maßnahmen erforderlich, sondern ist gegebenenfalls eine Maßnahme ausreichend.

Ein Umpositionieren des Leuchtenkörpers 2 der Operationsleuchte 1 wird durch einen nicht gezeigten Beschleunigungssensor, der in dem Leuchtenkörper 2 vorgesehen ist und mit der Steuerungsvorrichtung der Operationsleuchte 1 verbunden ist, erkannt. Die Bedieneinrichtung 7 ist so konfiguriert, dass sie bei dem Umpositionieren über eine Steuerungsvorrichtung der Operationsleuchte 1 oder alternativ durch den Beschleunigungssensor direkt angesteuert wird, nicht auf die Signale der vertikalen Sensoren 23 und/oder des horizontalen Sensors 24 zu reagieren, so dass keine Signale an die Fokussiereinrichtung 5 und/oder die Dimmvorrichtung 8 weitergegeben werden.

Ein Fokussieren der Lichtbündel 11 oder ein Dimmen der Helligkeit beginnt nur dann, wenn nach der Erkennung eines Objekts an der Handgriffaufnahme 6 auch eine Bewegung des Objekts erkannt wird, und ein bestimmter Weg, z.B. 1 cm, zurückgelegt wird. Nur eine bloße Anwesenheit eines Objekts, wie z.B. das Greifen des Handgriffs 14 zum Umpositionieren, wird nicht als Betätigung zum Verändern der Fokussierung oder zum Dimmen erkannt.

Das Leuchtmittel 16 wird verwendet, um eine Veränderung von Einstellungen der Operationsleuchte 1 anzuzeigen. Das Leuchtmittel 16 wird in einer Grundhelligkeit bei einer mittleren Helligkeit betrieben. Beim Verändern einer der verstellbaren Einstellungen der Operationsleuchte 1, zum Beispiel Dimmen oder Fokussieren, wird dies durch Verändern der Helligkeit des Anzeigeleuchtmittels 16 angezeigt. Durch die Ansteuerung der Dimmvorrichtung 8 zum Erhöhen der Leuchtstärke der Operationsleuchte 1 wird auch die Helligkeit des Anzeigeleuchtmittels erhöht. Auch eine Veränderung der Fokussierung wird angezeigt, indem bei einem Zusammenfahren der Randmodule 9 die Helligkeit des Anzeigeleuchtmittels 16 erhöht wird. Eine Reduzierung der Leuchtstärke des Anzeigeleuchtmittels 16 erfolgt jeweils durch umgekehrte Ansteuerung, also beim Reduzieren der Leuchtstärke der Operationsleuchte 1, oder beim Auseinander fahren der Randmodule 9. Nachdem der Einstellvorgang an der Bedieneinrichtung 7 abgeschlossen ist, wird das Anzeigeleuchtmittel 16 durch eine Steuerungseinheit, mit der es verbunden ist, wieder auf die Grundhelligkeit eingestellt, und bei einem darauf folgenden Verändern einer Einstellung wird dieses wieder relativ zu der Veränderung angezeigt.

Das Leuchtmittel 16 ist in Form einer LED ausgeführt. Auch ist alternativ eine Skala bestehend aus mehreren LEDs möglich. Weiterhin ist es alternativ möglich, die Anzeige entsprechend der momentan veränderten Funktion der Operationsleuchte 1 zu aktivieren und die aktuelle Einstellung anzuzeigen. Alternativ ist auch die Anzeige einer Einstellung, z.B. der Fokussierung, dauerhaft möglich. Darüber hinaus ist auch eine akustische Rückmeldung denkbar.

Die Anzahl der Sensoren 23, 24 ist abhängig von der Anzahl der einzustellenden Funktionen. Für eine ergonomische Bedienung der Operationsleuchte können auch mehrere Sensoren 23, 24 für eine Funktion an verschiedenen Stellen der Operationsleuchte vorgesehen sein, oder ein Sensor an einer gewünschten Stelle der Operationsleuchte. In jedem Fall werden die Sensoren aber durch eine sterilisierbare Abdeckung, z.B. den Hangriff 14, die den Sensor abdeckt, hindurch angesteuert.

## Patentansprüche

1. Operationsleuchte (1) mit
einem Leuchtenkörper (2), der mindestens eine Lichtquelle (3) aufweist, wobei aus dem Leuchtenköper (2) mindestens ein Lichtbündel (11) abgegeben wird, und
einer Bedieneinrichtung (7), die an der Operationsleuchte (1) angeordnet ist, **dadurch gekennzeichnet, dass**
eine Fokussiereinrichtung (5) in dem Leuchtenkörper zum Fokussieren des Lichtbündels (11) oder der Lichtbündel (11) vorgesehen ist, und dass
die Bedieneinrichtung (7) direkt oder indirekt mit der Fokussiereinrichtung (5) verbunden ist, und die Bedieneinrichtung (7) mit einer sterilisierbaren Abdeckung abdeckbar ist, wobei
die Bedieneinrichtung (7) angepasst ist, berührungslos einen vorbestimmten Bewegungsablauf von einem Bediener zu erkennen und daraufhin die Fokussiereinrichtung (5) anzusteuern, und wobei
die Bedieneinrichtung (7) mit der sterilisierbaren Abdeckung abgedeckt ist.

2. Operationsleuchte (1) gemäß Anspruch 1, wobei die Operationsleuchte (1) zusätzlich
eine Aufhängevorrichtung (4) aufweist, an der der Leuchtenkörper angebracht ist, und
eine Handgriffaufnahme (6) aufweist, die an der Aufhängevorrichtung (4) oder an dem Leuchtenkörper (2) zum Aufnehmen eines Handgriffs (14) angebracht ist,
**dadurch gekennzeichnet, dass**
die Bedieneinrichtung (7) an der Handgriffaufnahme (6) angeordnet ist, und die sterilisierbare Abdeckung der Handgriff (14) ist.

3. Operationsleuchte (1) gemäß Anspruch 1 oder 2, **dadurch gekennzeichnet, dass** zusätzlich eine Dimmvorrichtung (8) für die Lichtquelle (3) vorgesehen ist, und die Bedieneinrichtung (7) direkt oder indirekt mit der Dimmvorrichtung (8) verbunden ist, so dass eine Betätigung der Bedieneinrichtung (7) durch einen vorbestimmten Bewegungsablauf von einem Bediener ein Dimmen der Lichtquelle (3) ansteuert.

4. Operationsleuchte (1) gemäß Anspruch 3, **dadurch gekennzeichnet, dass** die Bedieneinrichtung (7) angepasst ist, dass sie verschiedene der Fokussiereinrichtung (5) und der Dimmvorrichtung (8) vorbestimmt zugeordnete Bewegungsabläufe des Bedieners erkennt und ein jeweiliges Ansteuerungssignal an die Fokussiereinrichtung (5) oder die Dimmvorrichtung (8) abgibt.

5. Operationsleuchte (1) gemäß Anspruch 4, **dadurch gekennzeichnet, dass** die Bedieneinrichtung (7) angepasst ist, dass ein Bewegungsablauf zum Ansteuern der Fokussiereinrichtung (5) unterschiedlich zu einem Bewegungsablauf zum Ansteuern der Dimmvorrichtung (8) ist.

6. Operationsleuchte (1) gemäß Anspruch 4 oder 5, **dadurch gekennzeichnet, dass** die Bedieneinrichtung (7) angepasst ist, dass die Bedieneinrichtung (7) verschiedene Richtungen des jeweiligen Bewegungsablaufs erkennt und ein jeweiliges Ansteuerungssignal an die Fokussiereinrichtung (5) oder die Dimmvorrichtung (8) abgibt.

7. Operationsleuchte (1) gemäß Anspruch 6, **dadurch gekennzeichnet, dass** der Leuchtenkörper (2) ein Leuchtmittel (16) als Anzeigevorrichtung aufweist, das angepasst ist, dass die Anzeigevorrichtung abhängig von der Richtung des Bewegungsablaufs ausgehend von einer Grundhelligkeit heller oder dunkler wird.

8. Operationsleuchte (1) gemäß Anspruch 7, **dadurch gekennzeichnet, dass** die Anzeigevorrichtung mit einer Steuerungseinheit verbunden ist, die angepasst ist, dass sie nach Abschluss des Bewegungsablaufs die Anzeigevorrichtung wieder auf die Grundhelligkeit einstellt.

9. Operationsleuchte (1) gemäß einem der vorangehenden Ansprüche, **dadurch gekennzeichnet, dass** die Bedieneinrichtung (7) eine Einrichtung zum Einstellen der Empfindlichkeit des Erkennens der berührungslosen Betätigung aufweist.

10. Operationsleuchte (1) gemäß Anspruch 9, **dadurch gekennzeichnet, dass** die Bedieneinrichtung (7) eine Einrichtung zum Kalibrieren der Empfindlichkeit des Erkennens der berührungslosen Betätigung aufweist.

11. Operationsleuchte (1) gemäß einem der vorangehenden Ansprüche, **dadurch gekennzeichnet, dass** die Operationsleuchte (1) einen Beschleunigungssensor aufweist, der mit der Bedieneinrichtung (7) direkt oder indirekt verbunden ist, und die Operationsleuchte (1) angepasst ist, dass die Fokussiereinrichtung (5) nicht angesteuert wird, wenn der Bewegungssensor eine Bewegung des Leuchtenkörpers (2) erfasst.

12. Operationsleuchte (1) gemäß einem der vorangehenden Ansprüche, **dadurch gekennzeichnet, dass** die Bedieneinrichtung (7) mindestens einen kapazitiven Sensor (19) aufweist.

13. Operationsleuchte (1) gemäß einem der vorangehenden Ansprüche, **dadurch gekennzeichnet, dass** die Bedieneinrichtung (7) so angepasst ist, dass die berührungslose Betätigung mit und ohne Handgriff (14) möglich ist.

## Claims

1. Surgical lamp (1) having
a lamp body (2) comprising at least one light source (3), wherein at least one bundle of light (11) is emitted out of the lamp body (2), and
an operating device (7) arranged on the surgical lamp (1),
**characterized in that**
a focusing device (5) in the lamp body for focusing the bundle of light (11) or the bundles of light (11) is provided, and that
the operating device (7) is directly or indirectly connected to the focusing device (5), and the operating device (7) is coverable by a sterilizable cover, wherein
the operating device (7) is adapted to recognize a predetermined motion sequence of an operator in a contactless manner and, as a result of that, to control the focusing device (5), and wherein
the operating device (7) is covered by the sterilizable cover.

2. Surgical lamp (1) according to claim 1, wherein the surgical lamp (1) additionally comprises
a suspension device (4) at which the lamp body is attached, and
a handle receptacle (6) attached to the suspension device (4) or to the lamp body (2) for receiving a handle (14),
**characterized in that**
the operating device (7) is arranged at the handle receptacle (6) and the sterilizable cover is the handle (14).

3. Surgical lamp (1) according to claim 1 or 2, **characterized in that** a dimming device (8) for the light source (3) is additionally provided, and that the operating device (7) is directly or indirectly connected to the dimming device (8) so that an operation of the operating device (7) by a predetermined motion sequence of an operator controls dimming of the light source (3).

4. Surgical lamp (1) according to claim 3, **characterized in that** the operating device (7) is adapted such that it recognizes different motion sequences of the operator assigned to the focusing device (5) and to the dimming device (8) in a predetermined manner and that it submits a respective control signal to the focusing device (5) or to the dimming device (8).

5. Surgical lamp (1) according to claim 4, **characterized in that** the operating device (7) is adapted such that a motion sequence for controlling the focusing device (5) is different from a motion sequence for controlling the dimming device (8).

6. Surgical lamp (1) according to claim 4 or 5, **characterized in that** the operating device (7) is adapted such that the operating device (7) recognizes different directions of the respective motion sequence and submits a respective control signal to the focusing device (5) or to the dimming device (8).

7. Surgical lamp (1) according to claim 6, **characterized in that** the lamp body (2) comprises an illuminant (16) as an indicator device which is adapted such that, starting from a basic brightness, the indicator device becomes lighter or darker dependent on the direction of the motion sequence.

8. Surgical lamp (1) according to claim 7, **characterized in that** the indicator device is connected to a control unit which is adapted such that, after termination of the motion sequence, the indicator device is reset to the basic brightness.

9. Surgical lamp (1) according to any of the preceding claims, **characterized in that** the operating device (7) comprises a device for adjusting the sensitivity of the recognition of the contactless operation.

10. Surgical lamp (1) according to claim 9, **characterized in that** the operating device (7) comprises a device for calibrating the sensitivity of the recognition of the contactless operation.

11. Surgical lamp (1) according to any of the preceding claims, **characterized in that** the surgical lamp (1) comprises an acceleration sensor being directly or indirectly connected to the operating device (7), and that the surgical lamp (1) is adapted such that the focusing device (5) is not actuated when the motion sensor detects a movement of the lamp body (2).

12. Surgical lamp (1) according to any of the preceding claims, **characterized in that** the operating device (7) comprises at least one capacitive sensor (19).

13. Surgical lamp (1) according to any of the preceding claims, **characterized in that** the operating device (7) is adapted such that the contactless operation is possible with and without a handle (14).

## Revendications

1. Lampe scialytique (1) comprenant :
un corps lumineux (2) comportant au moins une source de lumière (3), dans laquelle au moins un faisceau de lumière (11) est émis par le corps lumineux (2), et
un dispositif de manœuvre (7) installé sur la lampe scialytique (1),
**caractérisée en ce que**
un dispositif de focalisation (5) dans le corps lumineux pour focaliser le ou les faisceau(x) de lumière (11) est prévu, et que
le dispositif de manœuvre (7) est relié directement ou indirectement au dispositif de focalisation (5), et le dispositif de manœuvre (7) peut être recouvert par un recouvrement stérilisable, dans laquelle
le dispositif de manœuvre (7) est adapté pour identifier sans contact un mouvement prédéfini d'un utilisateur puis commander le dispositif de focalisation (5), et dans laquelle
le dispositif de manœuvre (7) est recouvert par un recouvrement stérilisable.

2. Lampe scialytique (1) selon la revendication 1, dans laquelle la lampe scialytique (1) comporte en outre
un dispositif de suspension (4) sur lequel est fixé le corps lumineux, et
un élément de réception d'une poignée (6) qui est fixé sur le dispositif de suspension (4) ou le corps lumineux (2) pour recevoir une poignée (14),
**caractérisée en ce que**
le dispositif de manœuvre (7) est monté sur l'élément de réception d'une poignée (6), et le recouvrement stérilisable est constitué par la poignée (14).

3. Lampe scialytique (1) selon la revendication 1 ou 2, **caractérisée en ce qu'**un dispositif variateur de luminosité (8) pour la source de lumière (3) est en outre prévu, et le dispositif de manœuvre (7) est relié directement ou indirectement au dispositif variateur de lumière (8) de sorte qu'un actionnement du dispositif de manœuvre (7) par un mouvement prédéfini d'un utilisateur commande une variation de la luminosité de la source de lumière (3).

4. Lampe scialytique (1) selon la revendication 3, **caractérisée en ce que** le dispositif de manœuvre (7) est adapté pour identifier différents mouvements de l'utilisateur associés de manière prédéfinie au dispositif de focalisation (5) et au dispositif variateur de luminosité (8) et délivrer un signal de commande respectif au dispositif de focalisation (5) ou au dispositif variateur de luminosité (8).

5. Lampe scialytique (1) selon la revendication 4, **caractérisée en ce que** le dispositif de manœuvre (7) est adapté de sorte qu'un mouvement permettant de commander le dispositif de focalisation (5) soit différent d'un mouvement permettant de commander le dispositif variateur de luminosité (8).

6. Lampe scialytique (1) selon la revendication 4 ou 5, **caractérisée en ce que** le dispositif de manœuvre (7) est adapté pour identifier différentes directions du mouvement respectif et délivrer un signal de commande respectif au dispositif de focalisation (5) ou au dispositif variateur de luminosité (8).

7. Lampe scialytique (1) selon la revendication 6, **caractérisée en ce que** le corps lumineux (2) comporte un moyen d'éclairage (16) constituant un dispositif indicateur qui est adapté, en partant d'une luminosité de base, pour devenir plus clair ou plus sombre en fonction de la direction du mouvement.

8. Lampe scialytique (1) selon la revendication 7, **caractérisée en ce que** le dispositif indicateur est relié à une unité de commande qui est adaptée pour régler à nouveau le dispositif indicateur sur la luminosité de base après achèvement du mouvement.

9. Lampe scialytique (1) selon l'une des revendications précédentes, **caractérisée en ce que** le dispositif de manœuvre (7) comporte un dispositif de réglage de la sensibilité de l'identification de l'actionnement sans contact.

10. Lampe scialytique (1) selon la revendication 9, **caractérisée en ce que** le dispositif de manœuvre (7) comporte un dispositif permettant de calibrer la sensibilité de l'identification de l'actionnement sans contact.

11. Lampe scialytique (1) selon l'une des revendications précédentes, **caractérisée en ce que** la lampe scialytique (1) comporte un capteur d'accélération qui est relié directement ou indirectement au dispositif de manœuvre (7), et la lampe scialytique (1) est adaptée de sorte que le dispositif de focalisation (5) ne soit pas commandé lorsque le capteur de mouvements détecte un mouvement du corps lumineux (2).

12. Lampe scialytique (1) selon l'une des revendications précédentes, **caractérisée en ce que** le dispositif de manœuvre (7) comporte au moins un capteur capacitif (19).

13. Lampe scialytique (1) selon l'une des revendications précédentes, **caractérisée en ce que** le dispositif de manœuvre (7) est adapté pour permettre un actionnement sans contact avec ou sans poignée (14).
